(19) Europäisches Patentamt / European Patent Office / Office européen des brevets

(11) **EP 3 909 940 B1**

(12) **EUROPÄISCHE PATENTSCHRIFT**

(45) Veröffentlichungstag und Bekanntmachung des Hinweises auf die Patenterteilung:
**22.02.2023 Patentblatt 2023/08**

(21) Anmeldenummer: **20174314.3**

(22) Anmeldetag: **13.05.2020**

(51) Internationale Patentklassifikation (IPC):
**C07C 2/10** *(2006.01)*   **C07C 7/04** *(2006.01)*
**C07C 11/02** *(2006.01)*   **C10G 50/00** *(2006.01)*

(52) Gemeinsame Patentklassifikation (CPC):
(C-Sets verfügbar)
**C07C 2/10; C07C 7/04; C10G 50/00;**
C07C 2521/12; C07C 2523/755   (Forts.)

(54) **VERFAHREN ZUR OLIGOMERISIERUNG VON OLEFINEN MIT KONTROLLE DES GEHALTS AN OLIGOMEREN IN DEN ZU OLIGOMERISIERENDEN KOHLENWASSERSTOFFSTRÖMEN**

METHOD FOR THE OLIGOMERISATION OF OLEFINS WITH CONTROL OF THE CONTENT OF OLIGOMERS IN HYDROCARBON STREAMS TO BE OLIGOMERISED

PROCÉDÉ D'OLIGOMÉRISATION D'OLÉFINES À CONTRÔLE DE LA TENEUR EN OLIGOMÈRES DANS LES FLUX D'HYDROCARBURES À OLIGOMÉRISER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.11.2021 Patentblatt 2021/46**

(73) Patentinhaber: **Evonik Operations GmbH**
**45128 Essen (DE)**

(72) Erfinder:
• **Stochniol, Guido**
**45721 Haltern am See (DE)**
• **Peitz, Stephan**
**45739 Oer-Erkenschwick (DE)**
• **Nadolny, Fabian**
**04157 Leipzig (DE)**
• **Berntsson, Benjamin William**
**40211 Düsseldorf (DE)**
• **Reeker, Helene**
**44227 Dortmund (DE)**

• **Bukohl, Reiner**
**45770 Marl (DE)**
• **Paul, Niklas**
**45770 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 1 457 475   WO-A1-2011/000697**

(52) Gemeinsame Patentklassifikation (CPC): (Forts.)

C-Sets
**C07C 2/10, C07C 11/02;**
**C07C 7/04, C07C 11/02**

C-Sets
**C07C 2/10, C07C 11/02;**
**C07C 7/04, C07C 11/02**

**Beschreibung**

Die vorliegende Erfindung betrifft ein Verfahren zur Oligomerisierung von C3- bis C5-Olefinen unter Verwendung eines Katalysators, wobei die Oligomerisierung in mindestens einer Reaktionsstufe, die mindestens einen Reaktor und mindestens eine Destillationskolonne umfasst, durchgeführt wird und wobei der Gehalt an Oligomeren im Zulaufstrom zur mindestens einen Reaktionsstufe nach Abtrennung der Oligomere in der mindestens einen Destillationskolonne kleiner als 0,4 Gew.-% ist.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen (Propen) ein Olefin mit sechs Kohlenstoffatomen (Hexen) aufbauen. Die Oligomerisierung von zwei Molekülen miteinander wird auch Dimerisierung genannt. Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Die Quelle für die Olefine für die Oligomerisierungsverfahren sind in der Regel olefinhaltige Fraktionen aus Crackverfahren (beispielweise Steamcracker oder Fluidkatalystische Cracker), die neben den Olefinen auch entsprechende Alkane enthalten.

Die Europäische Patentanmeldung EP1457475 A2 betrifft ein Verfahren zur Herstellung von Oligomeren von Alkenen mit 4 bis 8 Kohlenstoffatomen ausgehend von einem Feed-Strom aus solchen Alkenen oder solche Alkene enthaltenden Kohlenwasserstoffströmen an einem Nickel-haltigen, heterogenen Katalysator.

Nach der Herstellung der Olefinoligomere in einem oder mehreren hintereinandergeschalteten Reaktor(en) müssen die Oligomere vom Oligomerisat (Austrag aus der Oligomerisierung), welches unter anderem nicht umgesetzte Einsatzolefine und/oder Alkane enthält, abgetrennt werden. Die Abtrennung erfolgt typischerweise in mindestens einer Destillationskolonne, wobei die nicht umgesetzten Olefine und/oder die Alkane über Kopf gehen und zumindest teilweise zu dem oder den Reaktor(en) zurückgeführt werden.

Ziel der Abtrennung der Oligomere aus dem Oligomerisat ist üblicherweise, ein reines Oligomerisat ohne Eduktanteile und einen reinen Strom an nicht umgesetzten Einsatzolefinen und/oder Alkanen zu erzeugen, die dann entsprechend weiter verwertet werden. Das Problem ist jedoch, dass mit einer zu steigernden Reinheit der genannten Ströme auch die Kosten für den oder die Trennapparat(e) und/oder die Energiekosten, z.B. durch die Notwendigkeit eines erhöhten Kolonnenrücklaufes, deutlich höher sind. Zur Kostenreduktion werden die Oligomere deshalb üblicherweise nicht in reiner Form abgetrennt, sondern es verbleiben geringe Mengen an Edukten im abgetrennten Oligomerenstrom. Gleiches gilt für den Strom aus nicht umgesetzten Einsatzolefinen und/oder Alkanen, die auch nach der Abtrennung noch gewisse Mengen an Oligomeren enthalten.

Führt man diese Ströme aus Einsatzolefinen und/oder Alkanen sowie der Oligomere zurück zum Reaktor, fällt auf, dass einerseits die Oligomerausbeute sinkt und andererseits die Reaktionsrate in der Oligomerisierung abnimmt, je höher der rückgeführte Anteil an Oligomeren ist. Es ist also eine gewisse Inhibierung der Oligomerisierung zu berücksichtigen.

Die Aufgabe der vorliegenden Erfindung war deshalb die Bereitstellung eines Verfahrens zur Oligomerisierung von C3- bis C5-Olefinen, bei denen sich kein signifikantes Absinken der Reaktionsrate, also keine merkliche Inhibierung feststellen lässt und trotzdem eine kostengünstige Abtrennungswirkung erzielt werden kann.

Es konnte herausgefunden werden, dass die beschriebenen negativen Effekte (Absinken von Oligomerausbeute und Reaktionsrate) gerade bei einer nicht ausreichenden Abtrennung der Oligomere aus dem Strom der nicht umgesetzten Einsatzolefine und/oder der Alkane auftreten. Die zugrundeliegende Aufgabe konnte gemäß der vorliegenden Erfindung deshalb dadurch gelöst werden, dass der Gehalt an Oligomeren im Zulaufstrom zu dem oder den Reaktoren mindestens einer Reaktionsstufe kleiner als 0,4 Gew.-% ist, bezogen auf die Gesamtzusammensetzung des Zulaufs zu dem oder den Reaktoren der mindestens einen Reaktionsstufe ist. Der Zulauf besteht aus zurückgeführtem restlichen Oligomerisat, von dem die gebildeten Oligomere mittels Destillation abgetrennt worden sind, und dem Frischzulauf des Einsatzgemisches. Dies ist in Anspruch 1 wiedergegeben. Bevorzugte Ausgestaltungen des Verfahrens sind in den Unteransprüchen angegeben.

Das erfindungsgemäße Verfahren ist somit ein Verfahren zur Oligomerisierung von C3- bis C5-Olefinen, vorzugsweise C4-Olefinen wobei ein Einsatzgemisch, welches die C3- bis C5-Olefine, vorzugsweise die C4-Olefine enthält, unter Verwendung eines heterogenen Oligomerisierungskatalysators, der eine Nickelverbindung und ein Trägermaterial, enthaltend Aluminiumoxid, Siliciumdioxid oder Alumosilicat, umfasst, in mindestens einer Reaktionsstufe oligomerisiert wird, wobei eine Reaktionsstufe jeweils aus mindestens einem Reaktor, in dem die Oligomerisierung unter Bildung eines Oligomerisats durchgeführt wird, und mindestens einer Destillationskolonne besteht, in der die bei der Oligomerisierung gebildeten Oligomere zumindest teilweise vom restlichen Oligomerisat abgetrennt werden, wobei zumindest ein Teil des restlichen Oligomerisats, aus dem die Oligomere abgetrennt worden sind, zu einem oder mehreren Reaktor(en) der mindestens einen Reaktionsstufe zurückgeführt wird und der Gehalt an Oligomeren im Zulauf zu einem oder mehreren Reaktor(en) der mindestens einen Reaktionsstufe, welcher aus dem zurückgeführten restlichen Oligomerisat und dem Frischzulauf des Einsatzgemisches besteht, kleiner als 0,4 Gew.-%, vorzugsweise $\leq$ 0,2 Gew.-% ist, bezogen auf die Gesamtzusammensetzung des Zulaufs.

Der Gehalt an Oligomeren kann beispielsweise auch während des laufenden Betriebs mit gaschromatographischen Methoden überwacht werden. Um eine ausreichend Trennung zwischen Oligomeren und restlichem Oligomerisat und damit einen Gehalt an Oligomeren von kleiner als 0,4 Gew.-%, vorzugsweise $\leq$ 0,2 Gew.-% im Zulauf erreichen zu können, können verschiedene Maßnahmen einzeln oder in Kombination getroffen werden, beispielsweise die Verwendung einer oder mehrerer größerer Destillationskolonnen, bessere Packungen in der oder den Destillationskolonne(n), weniger Belastung auf die Destillationskolonne(n) oder einen erhöhten Destillatrücklauf in der oder den Destillationskolonne(n). Zudem lassen sich die erfindungsgemäßen Gehalte an Oligomeren auch durch die Einstellung des Verhältnisses von zurückgeführtem restlichen Oligomerisat zum Frischzulauf (zurückgeführtes restliches Oligomerisat / Frischzulauf) realisieren. Grundsätzlich können Oligomerengehalte von kleiner als 0,4 Gew.-%, vorzugsweise $\leq$ 0,2 Gew.-% auch dadurch realisiert werden, dass die Menge an zurückgeführtem restlichen Oligomerisat gering ist. In einer bevorzugten Ausführungsform beträgt das Verhältnis aus dem zurückgeführtem restlichen Oligomerisat (in t/h) und dem Frischzulauf (in t/h) 0,001 bis 30, vorzugsweise 0,005 bis 20 und besonders bevorzugt 0,01 bis 15.

Der Begriff "Reaktionsstufe" meint im Sinne der vorliegenden Erfindung einen Anlagenabschnitt, der einen oder mehreren Reaktor(en) und eine oder mehrere dem Reaktor nachfolgende Destillationskolonne(n) umfasst. In einer bevorzugten Ausführungsform ist nur eine Destillationskolonne pro Reaktionsstufe vorhanden. In den Destillationskolonnen werden die gebildeten Oligomere von dem Oligomerisat (entspricht dem Ausgangsstrom aus dem Reaktor), welches neben den Oligomeren auch Alkane und nicht umgesetzte Olefine umfasst, getrennt. Übliche verfahrenstechnische Aggregate, die in den Reaktionsstufen eingebaut sein können, wie beispielsweise Vorwärmer für den Feed, Wärmetauscher oder ähnliches, werden hier nicht separat aufgeführt, sondern sind dem Fachmann geläufig.

Das erfindungsgemäße Verfahren umfasst mindestens eine Reaktionsstufe. Das Verfahren kann aber auch mindestens zwei Reaktionsstufen umfassen, wobei vorzugsweise nicht mehr als fünf Reaktionsstufen vorliegen. In einer bevorzugten Ausführungsform umfasst das Verfahren zur Oligomerisierung daher zwei, drei, vier oder fünf Reaktionsstufen. Jede dieser Reaktionsstufen umfasst unabhängig voneinander einen oder mehreren Reaktoren und eine oder mehrere nachfolgende Destillationskolonnen, um die gebildeten Oligomere von dem restlichen Ausgangsstrom aus dem Reaktor abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Bei der einstufigen Verfahrensführung mit nur einer Reaktionsstufe werden die in dem Reaktor oder in den Reaktoren der ersten Reaktionsstufe gebildeten Oligomere in der Destillationskolonne der ersten Reaktionsstufe so vom restliche Oligomerisat abgetrennt, dass der Gehalt an Oligomeren im Gesamtzulauf zu dem oder den Reaktoren kleiner als 0,4 Gew.-%, bevorzugt $\leq$ 0,2 Gew.-% ist, wenn das restliche Oligomerisat zumindest teilweise mit dem Frischzulauf vermischt und zu dem oder den Reaktoren der Reaktionsstufe geführt wird. Bei der Verfahrensführung mit zwei oder mehr Reaktionsstufen ist der Gehalt an Oligomeren im Zulaufstrom für alle Reaktoren aller Reaktionsstufen kleiner als 0,4 Gew.-%, bevorzugt $\leq$ 0,2 Gew.-%. Bei der Verfahrensführung mit zwei oder mehr Reaktionsstufen kann das restliche Oligomerisat, von dem in der Destillationskolonne die Oligomere abgetrennt worden sind, teilweise zu dem oder zu einem der Reaktor(en) der gleichen Reaktionsstufe und teilweise zur nächsten Reaktionsstufe geführt werden. Die zumindest teilweise Weiterleitung des restlichen Oligomerisats an die nächste Reaktionsstufe entfällt bei der letzten Reaktionsstufe naturgemäß. Neben der Rückführung zum Reaktor der gleichen Reaktionsstufe und der Weiterleitung an die nächste Reaktionsstufe kann auch ein Teil des restlichen Oligomerisats entnommen werden, beispielsweise um zu verhindern, dass sich inerte Alkane im System anreichern.

Das erfindungsgemäße Verfahren kann ganz allgemein wie folgt durchgeführt werden: Ausgangspunkt ist die Bereitstellung eines Einsatzgemisches, welches C3- bis C5-Olefine, vorzugsweise C4-Olefine enthält. Das Einsatzgemisch wird zunächst in dem mindestens einen Reaktor der ersten Reaktionsstufe oligomerisiert und das erhaltene Oligomerisat zu einer Destillationskolonne geleitet, bei der die gebildeten Oligomere (vorzugsweise C6- bis C24-Olefine, besonders bevorzugt C8- bis C24-Olefine) als Sumpfprodukt von dem restlichen Oligomerisat, welches zumindest nicht umgesetzte Olefine und Alkane aus dem Einsatzgemisch enthält und welches als Kopfprodukt anfällt, getrennt. Je nach Reaktionsstufe wird das restliche Oligomerisat dann zumindest teilweise als Zulaufstrom zur jeweils nächsten Reaktionsstufe geleitet und teilweise zum Reaktor der gleichen Reaktionsstufe recycliert und vorher mit dem Frischzulauf aus frischen Einsatzgemisch bzw. mit dem an Oligomeren abgereichertem Oligomerisat der vorherigen Stufe kombiniert. In der letzten Reaktionsstufe wird das restliche Oligomerisat nach Abtrennung der Oligomere teilweise zu einem der Reaktoren zurückgeführt und zumindest teilweise aus dem Verfahren ausgeschleust. Wird das restliche Oligomerisat aus der letzten Reaktionsstufe aus dem hier beschriebenen Verfahren ausgeschleust, kann dies als Syntheserohstoff für weitere Verfahren (z. B. Hydroformylierung, C-Quelle für Lichtbogen bei der Acetylenherstellung), als Verbrennungsgas oder nach Vollhydrierung zu den Alkanen als Treibgas, als Kochgas o. ä. dienen.

Als Olefine für das erfindungsgemäße Verfahren werden C3- bis C5-Olefine, bevorzugt C4-Olefine oder darauf basierende Olefingemische, die auch Anteile an analogen Alkanen enthalten können, eingesetzt. Geeignete Olefine sind unter anderem $\alpha$-Olefine, n-Olefine und Cycloalkene, vorzugsweise n-Olefine. In einer bevorzugten Ausführungsform handelt es sich bei dem C4-Olefin um n-Buten.

Die Olefine werden üblicherweise nicht in reiner Form als Edukte eingesetzt, sondern in technisch verfügbaren Mischungen. Der in dieser Erfindung verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete C4-olefinhaltige Stoffströme sind insbesondere das Raffinat I, das Raffinat II und das Raffinat III.

Als Reaktor für die jeweiligen Reaktionsstufen können alle dem Fachmann bekannten Reaktoren eingesetzt werden, die für die Oligomerisierung geeignet sind, bspw. Rohrreaktoren, Rohrbündelreaktoren, Settler-Riser-Reaktoren, Slurry-Reaktoren. Bevorzugt sind Rohrreaktoren und/oder Rohrbündelreaktoren. Sofern eine Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in dergleichen Reaktionsstufe. Ebenso ist es möglich, dass die Reaktoren in den einzelnen Reaktionsstufen, sofern mehrere Reaktionsstufen vorliegen, gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Der eine oder die Reaktoren der einzelnen Reaktionsstufen enthalten jeweils einen Oligomerisierungskatalysator zur Durchführung der Oligomerisierung, insbesondere einen heterogenen Oligomerisierunsgkatalysator. Der Oligomerisierungskatalysator liegt dabei insbesondere in Form eines Granulats, eines Extrudats oder in Tablettenform vor.

Die (heterogenen) Oligomerisierungskatalysatoren in den einzelnen Reaktoren der Reaktionsstufen können jeweils unabhängig voneinander aus Übergangsmetall-haltigen Oligomerisierungskatalysatoren ausgewählt werden. Die Übergangsmetalle bzw. die entsprechend eingesetzten Übergangsmetallverbindungen sind dabei vorzugsweise auf einem Trägermaterial, enthaltend Aluminiumoxid, Siliciumdioxid oder Alumosilicat vorzugsweise einem Alumosilicat-Trägermaterial, angeordnet. Als Übergangsmetallverbindungen für die erfindungsgemäße eingesetzten Oligomerisierungskatalysatoren eignen sich insbesondere Verbindungen von Nickel, Cobalt, Chrom, Titan und Tantal. Bevorzugt sind Nickel- und Cobalt-Verbindungen, besonders bevorzugt sind Nickel-Verbindungen.

Gemäß der vorliegenden Erfindung umfasst der erfindungsgemäße Oligomerisierungskatalysator eine Nickelverbindung, vorzugsweise Nickeloxid, und ein Trägermaterial, welches Aluminiumoxid, Siliciumdioxid oder Alumosilicat, vorzugsweise Alumosilicat, enthält oder daraus besteht, eingesetzt werden. Das Trägermaterial ist vorzugsweise ein amorphes, mesoporöses Alumosilicat, ein kristallines, mikroporöses Alumosilicat oder ein Alumosilicat, welches amorphe und kristalline Phasen aufweist. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Alumosilicat kleine kristalline Domänen aufweist.

Erfindungsgemäß weiterhin bevorzugt weist der Oligomerisierungskatalysator eine Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_2$ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. In einer besonders bevorzugten Ausführungsform der vorliegenden Erfindung ist der Oligomerisierungskatalysator im Wesentlichen frei von Titandioxid und/oder Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumdioxid in seiner Gesamtzusammensetzung.

Der Oligomerisierungskatalysator weist vorzugsweise eine spezifische Oberfläche (berechnet nach BET) von 150 bis 700 m²/g, weiterhin bevorzugt von 190 bis 600 m²/g, besonders bevorzugt von 220 bis 550 m²/g aufweisen. Die BET-Oberfläche wird mittels Stickstoff-Physisorption gemäß DIN-ISO 9277 (Stand: 2014-01) gemessen.

Sollten mehrere Reaktoren in einer Reaktionsstufe oder in mehreren Reaktionsstufen vorhanden sein, liegen naturgemäß auch mehrere Oligomerisierungskatalysatoren vor. Die in den einzelnen Reaktoren in den Reaktionsstufen vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre ändern.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Die Oligomerisierung kann in jeder der vorhandenen Reaktionsstufen bei einer Temperatur im Bereich von 50 bis 200 °C, vorzugsweise 60 bis 180 °C, bevorzugt im Bereich von 60 bis 130°C durchgeführt wird. Der Druck kann jeder der vorhandenen Reaktionsstufen von 10 bis 70 bar, bevorzugt von 20 bis 55 bar betragen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (also 1 h⁻¹) und 190 h⁻¹, vorzugsweise zwischen 2 h⁻¹ und 35 h⁻¹, besonders bevorzugt zwischen 3 h⁻¹ und 25 h⁻¹.

Insbesondere bei Verwendung eines Katalysators, welcher ein Nickelverbindung, vorzugsweise Nickeloxid, auf einem Trägermaterial umfasst, beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel, wobei sich der Anteil der einzelnen Dimerfraktionen auf die Gesamtdimer-Fraktion bezieht:

$$\frac{\text{(einfach verzweigte Dimere (Gew.-\%) + 2 x zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das C9-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Beispiele

Beispiel 1 (erfindungsgemäß):

Die Oligomerisierung wurde in einem weitgehend isotherm betriebenen Rohreaktor mit folgenden Maßen nachgestellt: Länge 2.0 m, Innendurchmesser 6 mm. Der Reaktor war zur Thermostatisierung in einen Thermostaten eingehängt. Als Wärmeträger diente das Produkt Marlotherm der Firma Sasol. Als Katalysator wurden 12,6 g eines Materials verwendet, welches gemäß Beispiel 1 der WO2011/000697A1 hergestellt und nach Beispiel 4 derselben Veröffentlichung nachbehandelt worden war.

Die Reaktion wurde bei einem Absolutdruck von 30 bar und einer Temperatur von 80°C in der Flüssigphase durchgeführt. Als Frischzulauf zum Reaktor wurde 1 kg/h eines C4-Kohlenwasserstoffgemisches verwendet, enthaltend folgende Komponenten:

1-Buten 22,7 Gew.-%
2-Buten 58,4 Gew.-%
Isobuten 0,7 Gew.-%
Butane 18,1 Gew.-%
C8-Olefine 0,1 Gew.-%

Es ein Umsatz an C4-Olefinen von 43,9 % erzielt, wobei folgende Oligomerisatverteilung im Reaktoraustrag erzielt wurde:

C8-Olefine 83,9 %
C12-Olefine 12,5 %
C16+-Olefine 3,7 %

Dies entspricht einer Produktmenge von 34,4 g/h an C8-Olefinen.

Beispiel 2 (nicht erfindungsgemäß):

Die Oligomerisierung wurde in einem weitgehend isotherm betriebenen Rohreaktor mit folgenden Maßen nachgestellt: Länge 2.0 m, Innendurchmesser 6 mm. Der Reaktor war zur Thermostatisierung in einen Thermostaten eingehängt. Als Wärmeträger diente das Produkt Marlotherm der Firma Sasol. Als Katalysator wurden 12,6 g eines Materials verwendet, welches gemäß Beispiel 1 der WO2011/000697A1 hergestellt und nach Beispiel 4 derselben Veröffentlichung nachbehandelt worden war.

Die Reaktion wurde bei einem Absolutdruck von 30 bar und einer Temperatur von 80°C in der Flüssigphase durchgeführt. Als Frischzulauf zum Reaktor wurde 1 kg/h eines C4-Kohlenwasserstoffgemisches verwendet, enthaltend folgende Komponenten:

1-Buten 22,2 Gew.-%
2-Buten 57,9 Gew.-%
Isobuten 0,6 Gew.-%
Butane 18,2 Gew.-%
C8-Olefine 1,1 Gew.-%

Es wurde ein Umsatz an C4-Olefinen von 41,1 % erzielt, wobei folgende Oligomerisatverteilung im Reaktoraustrag erzielt wurden:

C8-Olefine 83,9 %
C12-Olefine 12,4 %
C16+-Olefine 3,8 %

Dies entspricht einer Produktmenge von 32,0 g/h an C8-Olefinen.

Es konnte gezeigt werden, dass eine Erhöhung des C8-Olefingehaltes im Zulauf zum Reaktor zu einer Verringerung des Umsatzes um 2,8 Prozentpunkte und zu einer Verringerung der produzierten Menge an C8-Oligomeren von 7% führt.

Beispiel 3 (erfindungsgemäß):

Die Oligomerisierung wurde in einem weitgehend adiabat betriebenen Rohreaktor ohne Thermostatisierung mit folgenden Maßen nachgestellt: Länge 2.0 m, Innendurchmesser 20,5 mm. Als Katalysator wurden 300 g eines Materials verwendet, welches gemäß Beispiel 1 der WO2011/000697A1 hergestellt und nach Beispiel 4 derselben Veröffentlichung nachbehandelt worden war.

Die Reaktion wurde bei einem Absolutdruck von 30 bar und einer Reaktorzulauftemperatur von 90°C in der Flüssigphase durchgeführt. Als Frischzulauf zum Reaktor wurden 1,75 kg/h eines C4-Kohlenwasserstoffgemisches verwendet, enthaltend folgende Komponenten:

1-Buten 35,8 Gew.-%
2-Buten 42,4 Gew.-%
Isobuten 0,9 Gew.-%
Butane 20,9 Gew.-%
C8-Olefine 0,0 Gew.-%

Es wurde ein Umsatz an C4-Olefinen von 31,7 % erzielt, wobei folgende Oligomerisatverteilung im Reaktoraustrag erzielt wurden:

C8-Olefine 85,1 %
C12-Olefine 12,6 %
C16+-Olefine 2,3 %

Beispiel 4 (erfindungsgemäß):

Die Bedingungen und der Reaktor entsprachen denen des Beispiels 3. Es wurde ein C4-Gemisch eingesetzt, dass folgende Zusammensetzung aufwies:

1-Buten 36,0 Gew.-%
2-Buten 43,8 Gew.-%
Isobuten 0,7 Gew.-%
Butane 19,3 Gew.-%
C8-Olefine 0,2 Gew.-% (entspricht 3,5 g/h)

Es wurde ein Umsatz an C4-Olefinen von 31,7 % erzielt, wobei folgende Oligomerisatverteilung im Reaktoraustrag erzielt wurden:

C8-Olefine 85,0 %
C12-Olefine 12,6 %
C16+-Olefine 2,4 %

Es konnte kein Umsatzrückgang gegenüber Beispiel 3 festgestellt werden.

Beispiel 5 (nicht erfindungsgemäß):

Die Bedingungen und der Reaktor entsprachen denen des Beispiels 3. Es wurde ein C4-Gemisch eingesetzt, dass folgende Zusammensetzung aufwies:

1-Buten 36,1 Gew.-%
2-Buten 44,1 Gew.-%
Isobuten 1,2 Gew.-%
Butane 18,0 Gew.-%
C8-Olefine 0,4 Gew.-% (entspricht 7 g/h)

Es wurde ein Umsatz an C4-Olefinen von 30,1 % erzielt, wobei folgende Oligomerisatverteilung im Reaktoraustrag erzielt wurden:

C8-Olefine 85,0 %

C12-Olefine 12,6 %
C16+-Olefine 2,4 %

Es wurde gegenüber Beispiel 3 ein leichter Umsatzrückgang beobachtet.

Beispiel 6 (nicht erfindungsgemäß):

Die Bedingungen und der Reaktor entsprachen denen des Beispiels 3. Es wurde ein C4-Gemisch eingesetzt, dass folgende Zusammensetzung aufwies:

1-Buten 34,3 Gew.-%
2-Buten 44,1 Gew.-%
Isobuten 1,1 Gew.-%
Butane 19,9 Gew.-%
C8-Olefine 0,7 Gew.-% (entspricht 12 g/h)

Es wurde ein Umsatz an C4-Olefinen von 28,8 % erzielt, wobei folgende Oligomerisatverteilung im Reaktoraustrag erzielt wurden:

C8-Olefine 85,1 %
C12-Olefine 12,6 %
C16+-Olefine 2,3 %

**Patentansprüche**

1. Verfahren zur Oligomerisierung von C3- bis C5-Olefinen, wobei ein Einsatzgemisch, welches die C3- bis C5-Olefine enthält, unter Verwendung eines heterogenen Oligomerisierungskatalysators, der eine Nickelverbindung und ein Trägermaterial, enthaltend Aluminiumoxid, Siliciumdioxid oder Alumosilicat, umfasst, in mindestens einer Reaktionsstufe oligomerisiert wird, wobei eine Reaktionsstufe jeweils aus mindestens einem Reaktor, in dem die Oligomerisierung unter Bildung eines Oligomerisats durchgeführt wird, und mindestens einer Destillationskolonne besteht, in der die bei der Oligomerisierung gebildeten Oligomere zumindest teilweise vom restlichen Oligomerisat abgetrennt werden,
**dadurch gekennzeichnet, dass** zumindest ein Teil des restlichen Oligomerisats, aus dem die Oligomere abgetrennt worden sind, zu einem oder mehreren Reaktor(en) der mindestens einen Reaktionsstufe zurückgeführt wird und der Gehalt an Oligomeren im Zulauf zu einem oder mehreren Reaktor(en) der mindestens einen Reaktionsstufe, welcher aus dem zurückgeführten restlichen Oligomerisat und dem Frischzulauf des Einsatzgemisches besteht, kleiner als 0,4 Gew.-% ist, bezogen auf die Gesamtzusammensetzung des Zulaufs.

2. Verfahren nach Anspruch 1, wobei der Gehalt an Oligomeren im Zulauf zu einem oder mehreren Reaktor(en) der mindestens einen Reaktionsstufe $\leq$ 0,2 Gew.-% ist, bezogen auf die Gesamtzusammensetzung des Zulaufs.

3. Verfahren nach Anspruch 1 oder 2, wobei das Verfahren zur Oligomerisierung in mindestens zwei Reaktionsstufen erfolgt, wobei die in dem Reaktor oder in den Reaktoren der ersten Reaktionsstufe gebildeten Oligomere in der Destillationskolonne der ersten Reaktionsstufe vom restlichen Oligomerisat abgetrennt werden und wobei das restliche Oligomerisat teilweise zu dem oder den Reaktor(en) der gleichen Reaktionsstufe und teilweise zu dem oder den Reaktoren der nächsten Reaktionsstufe geführt wird.

4. Verfahren nach einem der Ansprüche 1 bis 3, wobei das Trägermaterial des Oligomerisierungskatalysators ein Alumosilicat umfasst.

5. Verfahren nach Anspruch 4, wobei das Trägermaterial des Oligomerisierungskatalysators aus einem Alumosilicat besteht.

6. Verfahren nach Anspruch 4 oder 5, wobei der heterogene Oligomerisierungskatalysator eine Zusammensetzung von 15 bis 40 Gew.-% NiO, 5 bis 30 Gew.-% $Al_2O_3$, 55 bis 80 Gew.-% SiOz und 0,01 bis 2,5 Gew.-% eines Alkalimetalloxids aufweist.

**7.** Verfahren nach einem der Ansprüche 1 bis 6, wobei der Oligomerisierungskatalysator eine spezifische Oberfläche, berechnet nach BET, von 150 bis 700 m$^2$/g aufweist.

**8.** Verfahren nach einem der Ansprüche 1 bis 7, wobei die Oligomerisierung in jeder der vorhandenen Reaktionsstufen bei einer Temperatur im Bereich von 50 bis 200 °C, vorzugsweise 60 bis 180 °C, besonders bevorzugt im Bereich von 60 bis 130°C durchgeführt wird.

**9.** Verfahren nach einem der Ansprüche 1 bis 8, wobei der Druck bei der Oligomerisierung in jeder der vorhandenen Reaktionsstufen von 10 bis 70 bar, bevorzugt von 20 bis 55 bar beträgt.

**10.** Verfahren nach einem der Ansprüche 1 bis 9, wobei das Verfahren ein Verfahren zur Oligomerisierung von C4-Olefinen ist.

**11.** Verfahren nach einem der Ansprüche 1 bis 10, wobei die Oligomerisierung in jeder der mindestens einen Reaktionsstufe in flüssiger Phase durchgeführt wird.

**12.** Verfahren nach einem der Ansprüche 1 bis 11, wobei der Grad der Dimerisierung nach der Oligomerisierung bezogen auf das umgesetzte Edukt mindestens 60 % beträgt.

**13.** Verfahren nach einem der Ansprüche 1 bis 12, wobei die gewichtbasierte Raumgeschwindigkeit (WHSV) bei der Oligomerisierung von 1 g Reaktand pro g Katalysator und pro h, also 1 h$^{-1}$, bis 190 h$^{-1}$ beträgt.

**Claims**

**1.** Process for the oligomerization of C3- to C5-olefins, wherein a feed mixture comprising the C3- to C5-olefins is oligomerized in at least one reaction stage using a heterogeneous oligomerization catalyst comprising a nickel compound and a support material, containing aluminium oxide, silicon dioxide or aluminosilicate, wherein one reaction stage in each case consists of at least one reactor in which the oligomerization is carried out forming an oligomerizate, and at least one distillation column in which the oligomers formed during the oligomerization are at least partially separated from the residual oligomerizate, **characterized in that** at least a portion of the residual oligomerizate from which the oligomers have been separated is recycled to one or more reactor (s) of the at least one reaction stage and the content of oligomers in the feed to one or more reactor(s) of the at least one reaction stage, which consists of the recycled residual oligomerizate and the fresh feed of the feed mixture, is less than 0.4% by weight, based on the total composition of the feed.

**2.** Process according to Claim 1, wherein the content of oligomers in the feed to one or more reactor(s) of the at least one reaction stage is ≤ 0.2% by weight, based on the total composition of the feed.

**3.** Process according to Claim 1 or 2, wherein the process for oligomerization is carried out in at least two reaction stages, wherein the oligomers formed in the reactor or in the reactors of the first reaction stage are separated from the residual oligomerizate in the distillation column of the first reaction stage and wherein the residual oligomerizate is fed partly to the reactor(s) of the same reaction stage and partly to the reactor(s) of the next reaction stage.

**4.** Process according to any of Claims 1 to 3, wherein the support material of the oligomerization catalyst comprises an aluminosilicate.

**5.** Process according to Claim 4, wherein the support material of the oligomerization catalyst consists of an aluminosilicate.

**6.** Process according to Claim 4 or 5, wherein the heterogeneous oligomerization catalyst has a composition of 15 to 40% by weight NiO, 5 to 30% by weight Al$_2$O$_3$, 55 to 80% by weight SiO$_2$ and 0.01 to 2.5% by weight of an alkali metal oxide.

**7.** Process according to any of Claims 1 to 6, wherein the oligomerization catalyst has a specific surface, calculated according to BET, of 150 to 700 m$^2$/g.

**8.** Process according to any of Claims 1 to 7, wherein the oligomerization in each of the reaction stages present is carried out at a temperature in the range of 50 to 200°C, preferably 60 to 180°C, particularly preferably in the range of 60 to 130°C.

9. Process according to any of Claims 1 to 8, wherein the pressure in the oligomerization in each of the reaction stages present is 10 to 70 bar, preferably 20 to 55 bar.

10. Process according to any of Claims 1 to 9, wherein the process is a process for oligomerizing C4-olefins.

11. Process according to any of Claims 1 to 10, wherein the oligomerization in each of the at least one reaction stages is carried out in the liquid phase.

12. Process according to any of Claims 1 to 11, wherein the degree of dimerization after the oligomerization is at least 60% based on the converted reactant.

13. Process according to any of Claims 1 to 12, wherein the weight hourly space velocity (WHSV) during the oligomerization is from 1 g of reactant per g of catalyst and per h, i.e. 1 $h^{-1}$, to 190 $h^{-1}$.

**Revendications**

1. Procédé pour l'oligomérisation d'oléfines en $C_3$-$C_5$, dans lequel est oligomérisé un mélange de charge qui contient des oléfines en $C_3$ à $C_5$, avec utilisation d'un catalyseur d'oligomérisation hétérogène qui comprend un composé de nickel et une matière de support contenant de l'oxyde d'aluminium, du dioxyde de silicium ou un aluminosilicate, dans au moins un étage de réaction, un étage de réaction consistant respectivement en au moins un réacteur dans lequel est effectuée l'oligomérisation avec formation d'un produit d'oligomérisation, et en au moins une colonne de distillation dans laquelle les oligomères formés lors de l'oligomérisation sont au moins en partie séparés du produit d'oligomérisation restant,
**caractérisé en ce qu'**au moins une partie du produit d'oligomérisation restant, à partir duquel ont été séparés les oligomères, est renvoyé à un ou plusieurs réacteur(s) dudit au moins un étage de réaction et la teneur en oligomères du courant d'alimentation arrivant à un ou plusieurs réacteur(s) dudit au moins un étage de réaction, qui est constitué du produit d'oligomérisation restant recyclé et du courant d'alimentation neuf du mélange de charge, est inférieure à 0,4 % en poids, par rapport à la composition totale du courant d'alimentation.

2. Procédé selon la revendication 1, dans lequel la teneur en oligomères du courant d'alimentation arrivant à un ou plusieurs réacteur(s) dudit au moins un étage de réaction est ≤ 0,2 % en poids, par rapport à la composition totale du courant d'alimentation.

3. Procédé selon la revendication 1 ou 2, dans lequel le procédé pour l'oligomérisation s'effectue dans au moins deux étages de réaction, dans lequel les oligomères formés dans le réacteur ou dans les réacteurs du premier étage de réaction sont séparés du produit d'oligomérisation restant, dans la colonne de distillation du premier étage de réaction, et dans lequel le produit d'oligomérisation restant est envoyé en partie au ou aux réacteur(s) du même étage de réaction et en partie au ou aux réacteur(s) de l'étage de réaction suivant.

4. Procédé selon l'une quelconque des revendications 1 à 3, dans lequel la matière de support du catalyseur d'oligomérisation comprend un aluminosilicate.

5. Procédé selon la revendication 4, dans lequel la matière de support du catalyseur d'oligomérisation consiste en un aluminosilicate.

6. Procédé selon la revendication 4 ou 5, dans lequel le catalyseur d'oligomérisation hétérogène présente une composition de 15 à 40 % en poids de NiO, 5 à 30 % en poids d'$Al_2O_3$, 55 à 80 % en poids de $SiO_2$ et 0,01 à 2,5 % en poids d'un oxyde de métal alcalin.

7. Procédé selon l'une quelconque des revendications 1 à 6, dans lequel le catalyseur d'oligomérisation présente une surface spécifique, calculée selon BET, de 150 à 700 $m^2$/g.

8. Procédé selon l'une quelconque des revendications 1 à 7, dans lequel l'oligomérisation est effectuée, dans chacun des étages de réaction présents, à une température dans la plage de 50 à 200 °C, de préférence 60 à 180 °C, de façon particulièrement préférée dans la plage de 60 à 130 °C.

9. Procédé selon l'une quelconque des revendications 1 à 8, dans lequel la pression lors de l'oligomérisation dans chacun des étages de réaction présents vaut de 10 à 70 bars, de préférence de 20 à 55 bars.

10. Procédé selon l'une quelconque des revendications 1 à 9, dans lequel le procédé est un procédé pour l'oligomérisation d'oléfines en $C_4$.

11. Procédé selon l'une quelconque des revendications 1 à 10, dans lequel l'oligomérisation dans chacun desdits au moins un étage de réaction est effectuée en phase liquide.

12. Procédé selon l'une quelconque des revendications 1 à 11, dans lequel le degré de la dimérisation après l'oligomérisation, par rapport au produit de départ mis en réaction, est d'au moins 60 %.

13. Procédé selon l'une quelconque des revendications 1 à 12, dans lequel la vitesse spatiale en poids par poids (WHSV) lors de l'oligomérisation de 1 g de produit mis en réaction par g de catalyseur et par h, à savoir 1 $h^{-1}$, vaut jusqu'à 190 $h^{-1}$.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1457475 A2

- WO 2011000697 A1